(19) **Europäisches Patentamt**
**European Patent Office**
**Office européen des brevets**

(11) **EP 3 618 787 B1**

(12) **EUROPÄISCHE PATENTSCHRIFT**

(45) Veröffentlichungstag und Bekanntmachung des Hinweises auf die Patenterteilung:
**25.02.2026 Patentblatt 2026/09**

(21) Anmeldenummer: **18723777.1**

(22) Anmeldetag: **03.05.2018**

(51) Internationale Patentklassifikation (IPC):
***A61F 9/008*** (2006.01)

(52) Gemeinsame Patentklassifikation (CPC):
**A61F 9/00827;** A61F 9/00836; A61F 9/0084;
A61F 2009/00872; A61F 2009/0088;
A61F 2009/00882

(86) Internationale Anmeldenummer:
**PCT/EP2018/061337**

(87) Internationale Veröffentlichungsnummer:
**WO 2018/202771 (08.11.2018 Gazette 2018/45)**

(54) **NACHBEHANDLUNG BEI AUGENCHIRURGISCHER REFRAKTIONSKORREKTUR**

POST-TREATMENT IN REFRACTION CORRECTION DURING EYE SURGERY

POST-TRAITEMENT LORS D'UNE CORRECTION DE LA RÉFRACTION PAR CHIRURGIE DE L'OEIL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.05.2017 DE 102017207529**

(43) Veröffentlichungstag der Anmeldung:
**11.03.2020 Patentblatt 2020/11**

(73) Patentinhaber: **Carl Zeiss Meditec AG 07745 Jena (DE)**

(72) Erfinder:
• **SEDKY, Ahmed
Heliopolis
Cairo (EG)**
• **BISCHOFF, Mark
07745 Jena (DE)**
• **WOTTKE, Matthias
07745 Jena (DE)**

(74) Vertreter: **Rößner, Ulrike
Carl Zeiss AG
Patentabteilung Jena
Carl-Zeiss-Promenade 10
07745 Jena (DE)**

(56) Entgegenhaltungen:
**WO-A1-2016/144404　　US-A1- 2011 224 658**

**Beschreibung**

Die Erfindung bezieht sich auf eine Planungseinrichtung zum Erzeugen von Steuerdaten für eine Behandlungsvorrichtung, die mittels einer Lasereinrichtung zumindest eine Schnittfläche in der Hornhaut erzeugt. Die Erfindung bezieht sich weiter auf eine Behandlungsvorrichtung, die eine Planungseinrichtung der genannten Art aufweist.

Die Erfindung bezieht sich weiter auf ein Verfahren zum Erzeugen von Steuerdaten für eine Behandlungsvorrichtung, die mittels einer Lasereinrichtung zumindest eine Schnittfläche in der Hornhaut erzeugt.

Die Erfindung bezieht sich schließlich ebenso auf ein Verfahren zur Augenchirurgie, wobei mittels einer Behandlungsvorrichtung mit einer Lasereinrichtung zumindest eine Schnittfläche in der Hornhaut erzeugt wird.

Im Stand der Technik sind verschiedenste Behandlungsverfahren mit dem Ziel der Refraktionskorrektur am menschlichen Auge bekannt. Ziel der Operationsmethoden ist es dabei, die Hornhaut gezielt zu verändern, um so die Lichtbrechung im Auge zu beeinflussen. Hierfür werden mehrere Operationsmethoden eingesetzt. Am verbreitetsten ist gegenwärtig die sogenannte Laser-Insitu-Keratomileusis, die auch LASIK abgekürzt wird. Dabei wird zuerst eine Hornhaut-Lamelle von der Hornhautoberfläche einseitig gelöst und zur Seite geklappt. Das Lösen dieser Lamelle kann mittels eines mechanischen Mikrokeratoms erfolgen, oder auch mittels eines sogenannten Laserkeratoms, wie es z.B. von Intralase Corp., Irvine, USA, vertrieben wird. Nachdem die Lamelle gelöst und zur Seite geklappt wurde, ist bei der LASIK-Operation die Anwendung eines Excimer-Lasers vorgesehen, der das derart unter der Lamelle freigelegte Hornhautgewebe durch Ablation abträgt. Nachdem auf diese Art und Weise unter der Hornhautoberfläche liegendes Volumen verdampft wurde, wird die Hornhaut-Lamelle wieder auf den ursprünglichen Platz zurückgeklappt.

Die Anwendung eines Laserkeratoms zum Freilegen der Lamelle ist gegenüber einem mechanischen Messer vorteilhaft, da die die geometrische Präzision verbessert und die Häufigkeit klinisch relevanter Komplikationen verringert ist. Insbesondere kann die Lamelle mit sehr viel konstanterer Dicke hergestellt werden, wenn Laserstrahlung verwendet wird. Auch ist die Schnittkante präzise geformt, was die Gefahr für Heilungsstörungen durch diese auch nach der Operation verbleibende Grenzfläche mindert. Nachteilig bei diesem Verfahren ist allerdings, dass zwei unterschiedliche Behandlungsvorrichtungen verwendet werden müssen, zum einen nämlich das Laserkeratom zum Freilegen der Lamelle und zum anderen der das Hornhautgewebe verdampfende Laser.

Diese Nachteile sind behoben bei einem Verfahren, das jüngst durch die Carl Zeiss Meditec AG implementiert wurde und mit der Bezeichnung FLEX abgekürzt wird. Bei diesem Verfahren zur Lentikelextraktion wird mittels eines Kurzpulslasers, vorzugsweise eines Femtosekundenlasers in der Augenhornhaut eine Schnittgeometrie gebildet, welche in der Hornhaut ein Hornhaut-Volumen (sog. Lentikel) separiert. Dieses wird dann manuell vom Operateur entnommen, nachdem die das Lentikel bedeckende Lamelle zur Seite geklappt wurde. Der Vorteil dieses Verfahrens liegt zum einen darin, dass die Genauigkeit der Operation durch Anwendung des Femtosekundenlasers nochmals verbessert ist.

Zum anderen ist nur noch eine Behandlungsvorrichtung erforderlich; der Excimer-Laser wird nicht mehr eingesetzt.

Eine Weiterentwicklung des FLEX-Verfahrens wird in der Literatur als SMILE-Verfahren bezeichnet, bei dem kein Flap erzeugt wird, sondern nur ein kleiner Öffnungsschnitt als Zugang zu dem unter dem sogenannten Cap liegenden Lentikel dient. Das separierte Lentikel wird durch diesen kleinen Öffnungsschnitt entnommen, wodurch die biomechanische Integrität der vorderen Hornhaut weniger beeinträchtigt wird als bei LASIK, FLEX oder PRK (Photorefraktive Keratektomie). Hinzu kommt, dass auf diese Weise oberflächliche weniger Nervenfasern in der Hornhaut zerschnitten werden, was sich nachweislich günstig auf die Wiederherstellung der ursprünglichen Sensibilität der Hornhautoberfläche auswirkt. Das nach LASIK oft zu behandelnde Symptom trockener Augen ist dadurch in seiner Ausprägung und Dauer reduziert. Auch andere Komplikationen nach LASIK, die meist mit dem Flap im Zusammenhang stehen (z.B. Falten, Epithel-Einwachsungen im Flapbett) treten ohne Flap seltener auf.

Bei der Erzeugung von Schnittflächen in der Hornhaut mittels Laserstrahlung wird üblicherweise die optische Strahlungswirkung dadurch ausgenutzt, dass ein optischer Durchbruch durch einzelne optische Pulse, deren Dauer zwischen etwa 100 fs und 100 ns liegen kann, erzeugt wird. Auch ist es bekannt, einzelne Pulse, deren Energie unter einem Schwellwert für einen optischen Durchbruch liegt, derart überdeckt ins Gewebe bzw. Material einzubringen, dass auch damit eine Material- bzw. Gewebetrennung erreicht wird. Das Konzept der Schnitterzeugung im Hornhautgewebe mittels flächenhaft aneinandergereihter Fokusse (Fokuspunkte) ultrakurzer Laserpulse erlaubt eine große Vielfalt an Schnitten.

Es kann sich die Notwendigkeit ergeben, eine Nachbehandlung oder erneute Behandlung durchzuführen. Gründe dafür können z.B. sein, dass die ursprüngliche Behandlung abgebrochen werden musste oder auch dass sich die Refraktion des Auges so geändert hat, dass eine erneute Refraktionskorrektur notwendig wird.

Für das FLEX-Verfahren ist dazu im Stand der Technik eine Lösung bekannt (DE 10 2007 019814 A1, auf deren vollständigen Inhalt hiermit Bezug genommen wird). Diese beruht darauf, die neuen Schnitte so anzuordnen, dass sie die voroperativen Schnitte nicht schneiden. Diese Lösung ist auch für das SMILE-Verfahren grundsätzlich anwendbar. Es wurde in der DE 10 2012 022 081 A1, auf deren vollständigen Inhalt hiermit Bezug genommen wird, allerdings vorgeschlagen, die Lage der voroperativen Schnitte durch Messung zu bestimmen und diese bei einer verbesserten Korrektur zu berücksichtigen.

Es hat sich jedoch gezeigt, dass die Messung der vooperativen Schnitte mit einer ganzen Reihe praktischer Schwierigkeiten verbunden ist. Es besteht deshalb Grund zur Schlussfolgerung, dass die Korrekturverfahren nach dem Stand der Technik einerseits nicht immer optimale Ergebnisse gebracht haben und andererseits recht aufwendig sind. Eine Planungseinrichtung nach dem einleitenden Teil des Anspruchs 1 ist aus US2011224658A1 bekannt.

Der Erfindung liegt deshalb die Aufgabe zugrunde, eine Planungseinrichtung zum Erzeugen von Steuerdaten, eine Behandlungsvorrichtung zur Refraktion korrigierenden Augenchirurgie sowie ein Verfahren zum Erzeugen von Steuerdaten für eine solche Behandlungsvorrichtung anzugeben, welche eine verbesserte nachträgliche Refraktionskorrektur ermöglicht, die für Anwender des SMILE-Verfahrens auch mit derzeit verfügbaren Diagnosegeräten praktikabel ist.

Die Erfinder haben erkannt, dass durch die Entnahme eines Lentikels aus der Hornhaut beim SMILE-Verfahren eine Dickenänderung, insbesondere ein Dickenwachstum des Epithels der Hornhaut angeregt werden kann, welches bei der Planung der Nachbehandlung berücksichtigt werden sollte. Auf diese Weise kann im Unterschied zu früheren Methoden auf die mühsame und oft weniger genaue direkte Bestimmung der Lage vooperativer Schnitte ganz oder teilweise verzichtet werden.

Daher wird diese Aufgabe erfindungsgemäß mit einer Planungseinrichtung der eingangs genannten Art gelöst, die Berechnungsmittel zum Festlegen einer Hornhaut-Schnittfläche für eine Nachbehandlung aufweist, wobei die Berechnungsmittel so ausgebildet sind, dass eine Dickenänderung, insbesondere ein Dickenwachstum des Epithels bei der Berechnung berücksichtigt wird, die im Wesentlichen durch eine Vorbehandlung (Primärbehandlung) hervorgerufen wurde.

Die Erfindung wird weiter gelöst mit einer Behandlungsvorrichtung, die eine Lasereinrichtung aufweist, welche mittels Laserstrahlung gemäß Steuerdaten zumindest eine Schnittfläche in der Hornhaut trennt, und eine Planungseinrichtung nach der soeben genannten Art zum Erzeugen der Steuerdaten aufweist, wobei die Planungseinrichtung so ausgebildet ist, dass eine Dickenänderung, insbesondere ein Dickenwachstum des Epithels bei der Planung berücksichtigt wird.

Die Erfindung wird schließlich ebenfalls gelöst mit einem Verfahren zum Erzeugen von Steuerdaten gemäß der eingangs genannten Art, das aufweist: Bestimmung der Dickenänderung des Epithels, Erzeugen eines Steuerdatensatzes für die Hornhaut-Schnittfläche zur Ansteuerung der Lasereinrichtung, wobei die Dickenänderung des Epithels berücksichtigt wird.

Die Erfindung wird schließlich ebenfalls mit einem Verfahren gelöst, das umfasst: Bestimmung der Dickenänderung des Epithels, Erzeugen eines Steuerdatensatzes für die Hornhaut-Schnittfläche, Übertragen der Steuerdaten zur Behandlungsvorrichtung und Erzeugen der Schnittflächen durch Ansteuern der Lasereinrichtung mit dem Steuerdatensatz, wobei beim Erzeugen des Steuerdatensatzes die Dickenänderung des Epithels berücksichtigt wird.

Für die Messung der Dickenänderung des Epithels sind eine Reihe von Messvorrichtungen bzw. Messgeräten geeignet, z.B. OCT (Optical Coherence Tomography), Ultraschall, konfokales Laser Scanning System, Scheimpflug-Kamera oder andere. Wichtig ist, dass die Messgenauigkeit gleich oder besser als 2 $\mu$m ist. Die Messung kann unabhängig von der Nachbehandlung, also bereits als Grundlage einer vorausschauenden Planung erfolgen. Alternativ kann die Messung am Therapiegerät, mittels einer ins Therapiegerät integrierten Messvorrichtung erfolgen. Vorzugsweise ermöglicht die Messvorrichtung im unmittelbaren zeitlichen Zusammenhang mit der Nachbehandlung - quasi in Echtzeit - die Messung der Epitheldicke. Damit wird erreicht, dass Messgerät und Behandlungslaser geometrisch aufeinander abgeglichen sein können, also ein fester Koordinatenbezug der Diagnose- und Therapievorrichtung besteht.

Eine Variante der Bestimmung der Dickenänderung besteht in der vergleichenden Messung der Epitheldicke vor der primären Behandlung und vor der sekundären Behandlung.

Falls die Messung im Vorfeld erfolgen soll ist als zusätzlicher Schritt eine Zuordnung der Messwerte zum Koordinatensystem der Zielkoordinaten des Behandlungslasers vorzunehmen, wofür elektronische Datenübertragung und Registrierungslösungen vorteilhaft eingesetzt werden können.

Alternativ zu einer Messung kann das Epithelwachstum bzw. die Epitheldicke auch durch a-priori-Wissen, Abschätzung, Nomogramm oder ähnliches bestimmt werden. Dieses Wissen soll beispielsweise in der Planungseinrichtung als Programm-Code vorliegen. Insbesondere soll dann automatisch die typische Dickenänderung nach einer Erstbehandlung aus Information über die Erstbehandlung abgeleitet werden. Das ist insofern vorteilhaft, als die Abhängigkeit von unterschiedlichen Merkmalen des behandelten Auges (z.B. Alter, Größe, Refraktion) und der Erstbehandlung (Refraktionsänderung, Größe der Optischen Zone, Lage des Zugangsschnittes) für den Anwender einen erheblichen Aufwand bei der Vorhersage einer typischen Dickenänderung nach einer Erstbehandlung darstellt. Von noch größerer Bedeutung ist es aber, bei einer solchen medizinischen Behandlung das Risiko eines Planungsfehlers zu minimieren, wie es beispielsweise durch Verwechslungen von Größen oder Rechenfehler auftreten kann.

Die Dickenänderung $\Delta$Thickness ist zudem im Allgemeinen eine Funktion des Ortes auf der Oberfläche des zu behandelnden Auges, also $\Delta$Thickness(x,y) bzw. $\Delta$Thickness(r, $\phi$). Der Umgang mit einem solchen zweidimensionalen Profil und der korrekte Ortsbezug stellt für den Anwender eine Herausforderung dar, die durch entsprechendes Wissen der Planungseinrichtung in Form von Programm-Code wesentlich verringert werden kann.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:

Fig. 1 eine schematische Darstellung einer Behandlungsvorrichtung mit einer Planungseinrichtung für eine Behandlung bei augenchirurgischer Refraktionskorrektur,

Fig. 2 eine schematische Darstellung der Wirkung der Laserstrahlung, die in der Behandlungsvorrichtung der Fig. 1 verwendet wird,

Fig. 3 eine weitere Schemadarstellung des Behandlungsgerätes der Fig. 1 hinsichtlich der Einbringung der Laserstrahlung,

Fig. 4 eine schematische Schnittdarstellung durch die Augenhornhaut zur Veranschaulichung der Entnahme des Hornhaut-Volumens im Zusammenhang mit der augenchirurgischen Refraktionskorrektur,

Fig. 5 eine Schemadarstellung hinsichtlich des Aufbaus des Behandlungsgerätes der Fig. 1 mit besonderem Bezug auf die dort vorhandene Planungseinrichtung,

Fig. 6 eine Schemadarstellung einer Lentikelgeometrie SMILE mit den einzubringenden Schnitten gemäß dem Stand der Technik.

Fig. 7 eine Schemadarstellung hinsichtlich des Aufbaus des Behandlungsgerätes der Fig. 5 mit besonderem Bezug auf die dort vorhandene Messeinrichtung

Fig. 8 eine Schemadarstellung einer Lentikelgeometrie zur Korrektur gemäß der Erfindung

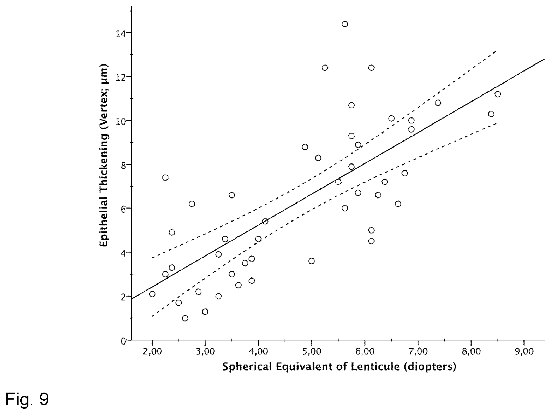

Fig. 9 eine Schemadarstellung der Abhängigkeit von Epitheldickenzunahme und Refraktionskorrektion

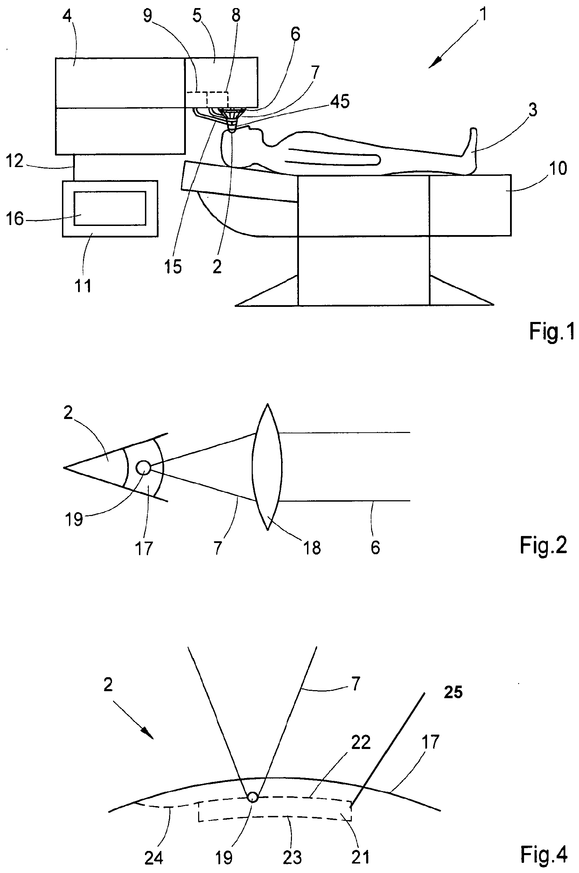

Eine Behandlungsvorrichtung für die Augenchirurgie ist in Fig. 1 dargestellt und mit dem allgemeinen Bezugszeichen 1 versehen. Die Behandlungsvorrichtung 1 ist für die Einbringung von Laserschnitten an einem Auge 2 eines Patienten 3 ausgebildet. Dazu weist die Behandlungsvorrichtung 1 eine Lasereinrichtung 4 auf, die aus einer Laserquelle 5 einen Laserstrahl 6 abgibt, welcher als fokussierter Strahl 7 in das Auge 2 bzw. die Augenhornhaut gerichtet wird. Vorzugsweise ist der Laserstrahl 6 ein gepulster Laserstrahl mit einer Wellenlänge zwischen 300 Nanometer und 10 Mikrometer. Weiter liegt die Pulslänge des Laserstrahls 6 im Bereich zwischen 1 Femtosekunde und 100 Nanosekunden, wobei Pulswiederholraten von 200 bis 20000 Kilohertz und Pulsenergien zwischen 0,01 Mikrojoule und 0,01 Millijoule möglich sind. Die Behandlungsvorrichtung 1 erzeugt somit in der Hornhaut des Auges 2 durch Ablenkung der gepulsten Laserstrahlung eine Schnittfläche. In der Lasereinrichtung 4 bzw. deren Laserquelle 5 ist deshalb dazu noch ein Scanner 8 sowie ein Strahlungsintensitätsmodulator 9 vorgesehen.

Der Patient 3 befindet sich auf einer Liege 10, die in drei Raumrichtungen verstellbar ist, um das Auge 2 passend zum Einfall des Laserstrahls 6 auszurichten. In bevorzugter Bauweise ist die Liege 10 motorisch verstellbar. Alternativ kann auch die Behandlungsvorrichtung verschoben werden.

Die Ansteuerung kann insbesondere durch ein Steuergerät 11 erfolgen, das grundsätzlich den Betrieb der Behandlungsvorrichtung 1 steuert und dazu über geeignete Datenverbindungen, beispielsweise Verbindungsleitungen 12 mit der Behandlungsvorrichtung verbunden ist. Natürlich kann diese Kommunikation auch über andere Wege, beispielsweise Lichtleiter oder per Funk geschehen. Das Steuergerät 11 nimmt die entsprechenden Einstellungen, Zeitsteuerung an der Behandlungsvorrichtung 1, insbesondere der Lasereinrichtung 4 vor und bewerkstelligt damit entsprechende Funktionen der Behandlungsvorrichtung 1.

Die Behandlungsvorrichtung 1 weist weiter noch eine Fixiereinrichtung 15 auf, welche die Hornhaut des Auges 2 gegenüber der Lasereinrichtung 4 lagefixiert. Diese Fixiereinrichtung 15 kann dabei ein bekanntes Kontaktglas 45 umfassen, an das die Augenhornhaut durch Unterdruck angelegt wird und das der Augenhornhaut eine gewünschte geometrische Form verleiht. Solche Kontaktgläser sind dem Fachmann aus dem Stand der Technik bekannt, beispielsweise aus der DE 10 2005 040338 A1. Der Offenbarungsgehalt dieser Druckschrift wird, soweit die Beschreibung einer Bauform des für die Behandlungsvorrichtung 1 möglichen Kontaktglases 45 betroffen ist, hier vollumfänglich einbezogen.

Die Behandlungseinrichtung 1 weist weiterhin eine hier nicht dargestellte Kamera auf, welche durch das Kontaktglas 45 hindurch ein Bild der Augenhornhaut 17 aufnehmen kann. Dabei kann die Beleuchtung für die Kamera sowohl im sichtbaren als auch im infraroten Bereich des Lichtes erfolgen.

Das Steuergerät 11 der Behandlungsvorrichtung 1 weist weiter noch eine Planungseinrichtung 16 auf, die später noch näher erläutert werden wird.

Fig. 2 zeigt schematisch die Wirkungsweise des einfallenden Laserstrahls 6. Der Laserstrahl 6 wird fokussiert und fällt als der fokussierte Laserstrahl 7 in die Hornhaut 17 des Auges 2. Zur Fokussierung ist eine schematisch eingezeichnete Optik 18 vorgesehen. Sie bewirkt in der Hornhaut 17 einen Fokus, in dem die Laserstrahlungsenergiedichte so hoch ist, dass in Kombination mit der Pulslänge der gepulsten Laserstrahlung 6 ein nicht-linearer Effekt in der Hornhaut 17 auftritt. Beispielsweise kann jeder Puls der gepulsten Laserstrahlung 6 im Fokus 19 einen optischen Durchbruch in der Augenhornhaut 17 erzeugen, welche wiederum eine in Fig. 2 nur schematisch angedeutete Plasmablase initiiert. Bei Entstehung der Plasmablase umfasst die Gewebsschichttrennung ein größeres Gebiet als den Fokus 19, obwohl die Bedingungen zur Erzeugung des optischen Durchbruches nur im Fokus 19 erreicht werden. Damit von jedem Laserpuls ein optischer Durchbruch erzeugt wird, muss die Energiedichte, d.h. die Fluence der Laserstrahlung oberhalb eines gewissen, pulslängenabhängigen Schwellwertes liegen. Dieser Zusammenhang ist dem Fachmann beispielsweise aus der DE 69500997 T2 bekannt. Alternativ kann ein gewebetrennender Effekt auch durch gepulste Laserstrahlung erreicht werden, indem mehrere Laserstrahlungspulse in einem Bereich abgegeben werden, wobei sich die Fokus-Spots überlappen. Es wirken dann mehrere Laserstrahlungspulse zusammen, um einen gewebetrennenden Effekt zu erreichen. Die Art der Gewebetrennung, die die Behandlungsvorrichtung 1 einsetzt, ist jedoch für die nachfolgende Beschreibung nicht weiter relevant; wesentlich ist lediglich, dass eine Schnittflächenerzeugung in der Hornhaut 17 des Auges 2 stattfindet.

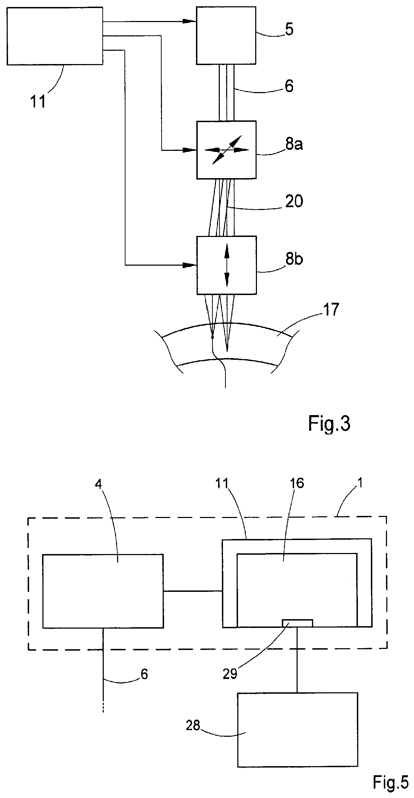

Um eine augenchirurgische Refraktionskorrektur auszuführen, wird mittels der Laserstrahlung 6 aus einem Gebiet innerhalb der Hornhaut 17 ein Hornhautvolumen entfernt, indem dort Gewebeschichten getrennt werden, die das Hornhaut-Volumen isolieren und dann dessen Entnahme ermöglichen. Zur Isolierung des zu entfernenden Hornhaut-Volumens wird z.B. im Falle der gepulst eingebrachten Laserstrahlung die Lage des Fokus 17 der fokussierten Laserstrahlung 7 in der Hornhaut 17 verstellt. Dies ist schematisch in Fig. 3 gezeigt. Die Brechungseigenschaften der Hornhaut 17 werden durch die Entnahme des Volumens gezielt verändert, um so die Refraktionskorrektur zu erreichen. Das Volumen ist deshalb meist linsenförmig und wird als Lentikel bezeichnet.

In Fig. 3 sind die Elemente der Behandlungsvorrichtung 1 nur insoweit eingetragen, als sie zum Verständnis der Schnittflächenerzeugung erforderlich sind. Der Laserstrahl 6 wird, wie bereits erwähnt, in einem Fokus 19 in der Hornhaut 19 gebündelt, und die Lage des Fokus 19 in der Hornhaut wird verstellt, so dass zur Schnittflächenerzeugung an verschiedenen Stellen fokussierende Energie aus Laserstrahlungspulsen in das Gewebe der Hornhaut 17 eingetragen wird. Die Laserstrahlung 6 wird von der Laserquelle 5 vorzugsweise als gepulste Strahlung bereitgestellt. Der Scanner 8 ist in der Bauweise der Fig. 3 zweiteilig aufgebaut und besteht aus einem xy-Scanner 8a, der in einer Variante durch zwei im Wesentlichen orthogonal ablenkende Galvanometerspiegel realisiert ist. Der Scanner 8a lenkt den von der Laserquelle 5 kommenden Laserstrahl 6 zweidimensional ab, so dass nach dem Scanner 9 ein abgelenkter Laserstrahl 20 vorliegt. Der Scanner 8a bewirkt somit eine Verstellung der Lage des Fokus 19 im Wesentlichen senkrecht zur Haupteinfallsrichtung des Laserstrahls 6 in der Hornhaut 17. Zur Verstellung der Tiefenlage ist neben dem xy-Scanner 8a im Scanner 8 noch ein z-Scanner 8b vorgesehen, der beispielsweise als verstellbares Teleskop ausgebildet ist. Der z-Scanner 8b sorgt dafür, dass die z-Position der Lage des Fokus 19, d.h. dessen Position auf der optischen Achse des Einfalls verändert wird. Der z-Scanner 8b kann dem xy-Scanner 8a nach- oder vorgeordnet sein.

Für das Funktionsprinzip der Behandlungsvorrichtung 1 ist die Zuordnung der einzelnen Koordinaten zu dem Raumrichtungen nicht wesentlich, genau so wenig, dass der Scanner 8a um zueinander rechtwinklige Achsen ablenkt. Vielmehr kann jeder Scanner verwendet werden, der in der Lage ist, den Fokus 19 in einer Ebene zu verstellen, in der die Einfallsachse der optischen Strahlung nicht liegt. Weiter können auch beliebige nichtkartesische Koordinatensystem zur Ablenkung bzw. Steuerung der Lage des Fokus 19 verwendet werden. Beispiele dafür sind Kugelkoordinaten oder zylindrische Koordinaten. Die Steuerung der Lage des Fokus 19 erfolgt mittels der Scanner 8a, 8b unter Ansteuerung durch das Steuergerät 11, das entsprechende Einstellungen an der Laserquelle 5, dem (in Fig. 3 nicht gezeigten) Modulator 9 sowie dem Scanner 8 vornimmt. Das Steuergerät 11 sorgt für einen geeigneten Betrieb der Laserquelle 5 sowie die hier exemplarisch geschilderte dreidimensionale Fokusverstellung, so dass letztendlich eine Schnittfläche ausgebildet wird, die ein bestimmtes Hornhaut-Volumen isoliert, das zur Refraktionskorrektur entfernt werden soll.

Die Steuereinrichtung 11 arbeitet nach vorgegebenen Steuerdaten, welche beispielsweise bei der hier lediglich exemplarisch geschilderten Lasereinrichtung 4 als Zielpunkte für die Fokusverstellung vorgegeben sind. Die Steuerdaten sind in der Regel in einem Steuerdatensatz zusammengefasst. Dies ergibt geometrische Vorgaben für die auszubildende Schnittfläche, beispielsweise die Koordinaten der Zielpunkte als Muster vor. Der Steuerdatensatz enthält dann in dieser Ausführungsform auch konkrete Stellenwerte für den Fokuslagenverstellmechanismus, z.B. für den Scanner 8.

Die Erzeugung der Schnittfläche mit der Behandlungsvorrichtung 1 ist exemplarisch in Fig. 4 gezeigt. Ein Hornhaut-Volumen 21 in der Hornhaut 17 wird durch Verstellung des Fokus 19, in dem der fokussierte Strahl 7 gebündelt ist, isoliert. Dazu werden Schnittflächen ausgebildet, die hier exemplarisch als anteriore Flap-Schnittfläche 22 sowie als posteriore Lentikel-Schnittfläche 23 ausgebildet sind. Diese Begriffe sind hier lediglich exemplarisch zu verstehen und sollen den Bezug auf das herkömmliche LASIK- oder FLEX-Verfahren herstellen, für das die Behandlungsvorrichtung 1, wie bereits geschildert, ebenfalls ausgebildet ist. Wesentlich ist hier lediglich, dass die Schnittflächen 22 und 23 sowie der umlaufende Randschnitt 25, welcher die Schnittflächen 22 und 23 an deren Rändern zusammenführen, das Hornhaut-Volumen 21 isolieren. Durch einen Öffnungsschnitt 24 kann weiter eine das Hornhaut-Volumen 21 anterior begrenzende Hornhaut-Lamelle abgeklappt werden, so dass das Hornhaut-Volumen 21 entnehmbar ist.

Alternativ und für die vorliegende Erfindung wesentlich kann das SMILE-Verfahren eingesetzt werden, bei der das Hornhautvolumen 21 durch einen kleinen Öffnungsschnitt entnommen wird, wie das in der DE 10 2007 019813 A1 beschrieben ist. Der Offenbarungsgehalt dieser Druckschrift wird hier vollumfänglich einbezogen

Fig. 5 zeigt schematisch die Behandlungsvorrichtung 1, und anhand ihr soll die Bedeutung der Planungseinrichtung 16 näher erläutert werden. Die Behandlungsvorrichtung 1 weist in dieser Variante mindestens zwei Einrichtungen oder Module auf. Die bereits geschilderte Lasereinrichtung 4 gibt den Laserstrahl 6 auf das Auge 2 ab. Der Betrieb der Lasereinrichtung 4 erfolgt dabei, wie bereits geschildert, voll automatisch durch das Steuergerät 11, d.h. die Lasereinrichtung 4 startet auf ein entsprechendes Startsignal hin die Erzeugung und Ablenkung des Laserstrahls 6 und erzeugt dabei Schnittflächen, die auf die beschriebene Art und Weise aufgebaut sind,. Die für den Betrieb erforderlichen Steuersignale empfängt die Lasereinrichtung 4 vom Steuergerät 11, dem zuvor entsprechende Steuerdaten bereitgestellt wurden. Dies erfolgt mittels der Planungseinrichtung 16, die in Fig. 5 lediglich exemplarisch als Bestandteil des Steuergeräts 11 gezeigt ist. Natürlich kann die Planungseinrichtung 16 auch eigenständig ausgebildet sein und drahtgebunden oder drahtlos mit der Steuereinrichtung 11 kommunizieren. Wesentlich ist dann lediglich, dass ein entsprechender Datenübertragungskanal zwischen der Planungseinrichtung 16 und dem Steuergerät 11 vorgesehen ist.

Die Planungseinrichtung 16 erzeugt einen Steuerdatensatz, der dem Steuergerät 11 zur Ausführung der augenchirurgischen Refraktionskorrektur zur Verfügung gestellt wird. Dabei verwendet die Planungseinrichtung Messdaten über die Hornhaut des Auges. Diese Daten stammen in der hier beschriebenen Ausführungsform aus einer Messeinrichtung 28, die das Auge 2 des Patienten 3 zuvor vermessen hat. Natürlich kann die Messeinrichtung 28 auf beliebige Art und Weise ausgebildet sein und die entsprechenden Daten an die Schnittstelle 29 der Planungseinrichtung 16 übermitteln. Insbesondere ist die Messeinrichtung 28 als OCT oder Ultraschall-Messsystem oder konfokales Laserscanningsystem ausgebildet um die Messdaten mit der erforderlichen Genauigkeit bereitzustellen.

Die Planungseinrichtung unterstützt nun den Bediener der Behandlungsvorrichtung 1 bei der Festlegung der Schnittfläche zur Isolierung des Hornhaut-Volumens 21. Dies kann bis zu einer vollautomatischen Festlegung der Schnittflächen gehen, die beispielsweise dadurch bewirkt werden kann, dass die Planungseinrichtung 16 aus den Messdaten das zu entnehmende Hornhaut-Volumen 21 ermittelt, dessen Begrenzungsflächen als Schnittflächen definiert und daraus entsprechende Steuerdaten für das Steuergerät 11 erzeugt. Am anderen Ende des Automatisierungsgrades kann die Planungseinrichtung 16 Eingabemöglichkeiten vorsehen, an denen ein Benutzer die Schnittflächen in Form von geometrischen Parametern etc. eingibt. Zwischenstufen sehen Vorschläge für die Schnittflächen vor, welche die Planungseinrichtung 16 automatisch generiert und die von einem Bearbeiter dann modifizierbar sind. Grundsätzlich können all diejenigen Konzepte, die im vorstehend allgemeineren Beschreibungsteil bereits erläutert wurden, hier in der Planungseinrichtung 16 zur Anwendung kommen.

Um eine Behandlung durchzuführen, erzeugt die Planungseinrichtung 16 Steuerdaten für die Schnittflächenerzeugung, die dann in der Behandlungsvorrichtung 1 verwendet werden.

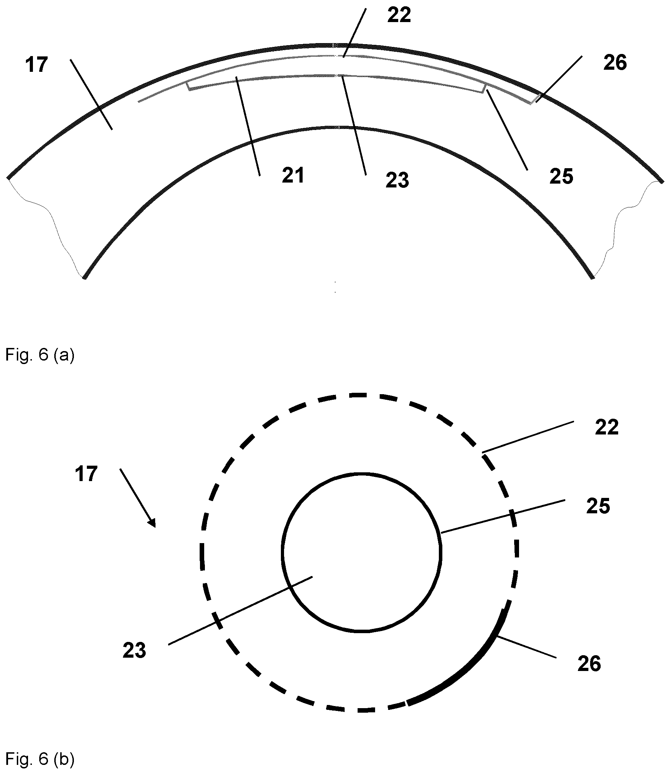

Fig. 6a zeigt eine Schemadarstellung eines Hornhautquerschnitts beim SMILE-Verfahren zur Verdeutlichung der geometrischen Verhältnisse. Die Hornhaut 17 weist einen anterioren Cap-Schnitt 22 mit einem Öffnungsschnitt 26 auf. Der posteriore Lentikelschnitt 23 isolierte das Lentikelvolumen 21, welches durch den Öffnungsschnitt 26 entnommen werden kann. Wenn sich jetzt die Notwendigkeit ergibt, eine zweite refraktive Behandlung durchzuführen, ist ein neues Lentikel zu berechnen. Dazu muss die Lage und Form des Cap-Schnitts 22 der ursprünglichen Behandlung mit der erforderlichen Genauigkeit bestimmt werden. Dies kann in einer ersten Variante der Erfindung durch eine Messung mit der Messeinrichtung 28 erfolgen. Die Art und Weise einer solchen Messung ist im Prinzip aus der DE 103 23 422 A1 bekannt, auf welche hiermit vollinhaltlich Bezug genommen wird. In dieser Schrift dient die Messeinrichtung allerdings einem anderen Zweck, nämlich der Selbstkalibrierung des Behandlungslasers während der Behandlung um die erforderliche Genauigkeit (besser als 5 $\mu$m) zu gewährleisten. Das Prinzip der Ultraschallmessung ist in "Refractive Surface Ablation: PRK, LASEK, Epi-lasik, Custom, PTK, and Retreatment" von Paolo Vinciquerra, Fabrizio Camesasca - Chapter 36 Dan Z. Reinstein, Ronald H. Silverman, Timothy J. Archer - "Very high frequency digital ultrasound: artemis 2 scanning in corneal refractive surgery", SLACK Inc. Thorofare, NJ, USA, 2007 erläutert, die erreichte Messgenauigkeit beträgt 1$\mu$m und ist daher für die vorliegende Aufgabe geeignet. Aus der aktuellen Lage des Capschnitts 22 und dem vorliegenden Bedarf der refraktiven Korrektur wird in der Planungseinrichtung 16 eine neue Lentikelschnittfläche 23 und ein neuer Randschnitt 25 berechnet und mittels der Behandlungsvorrichtung 1 in der Hornhaut 17 geschnitten.

Die Lage und Form der Referenzfläche in Bezug zur Cornea-Vorderseite, d.h. die Tiefe des ursprünglichen Capschnitts 22 kann sich postoperativ durch Epithelisierung (epitheliale Hyperplasie), Veränderung des Tränenfilms, Schwellungen der Cornea, Ödeme in der Cornea oder ähnliches ändern. Insbesondere verursacht eine Dickenänderung der Epithelschicht ΔThickness(x,y) bzw. ΔThickness(r, φ) eine entsprechende Änderung der relativen Lage des ursprünglichen Capschnitts bezüglich der Hornhautoberfläche.

Veränderungen wie Schwellungen, Änderungen des Tränenfilms u.a. können auch bei einem Abbruch der Operation (z.B. durch Verlust des Unterdrucks am Kontaktglas) auftreten und müssen ebenfalls berücksichtigt werden.

In einer ersten Variante der Erfindung werden die Veränderungen (Änderung der Epitheldicke infolge der primären Behandlung, relative Veränderung der Lage der bei der primären Behandlung eingebrachten Schnitte z.B. durch Epitheliale Hyperplasie) mit der Messeinrichtung 28 gemessen und fließen in die Berechnung der neuen Lentikelschnittfläche 23 ein.

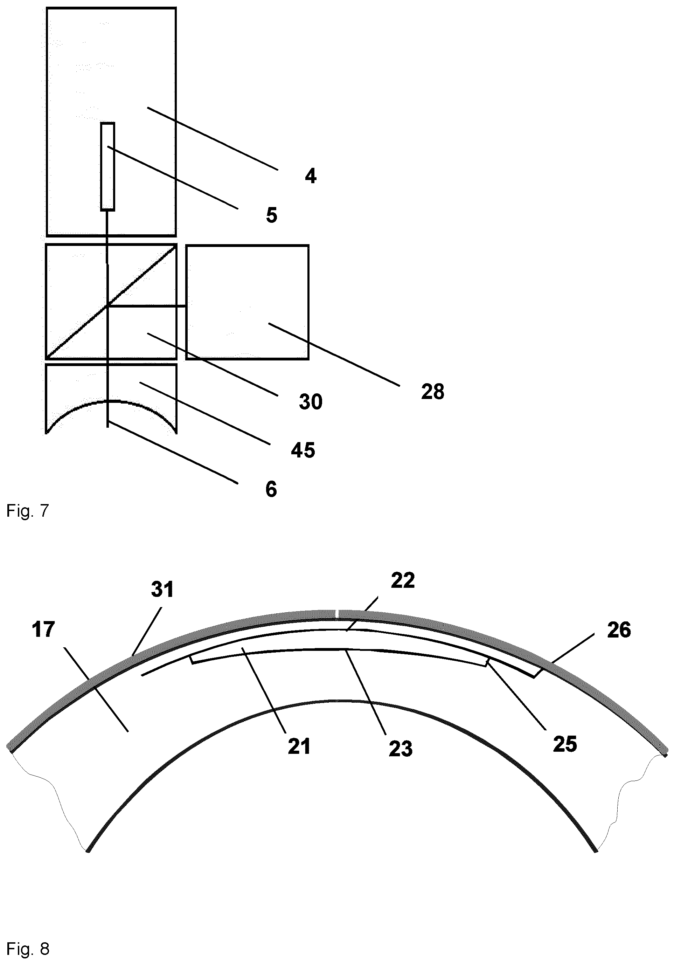

Fig. 7 zeigt eine Schemadarstellung des Aufbaus des Behandlungsgerätes der Fig. 5 und die Ankopplung der Messeinrichtung 28. Die Lasereinrichtung 4 weist eine Laserquelle 5 auf, welche einen Laserstrahl 6 erzeugt. Ein Kontaktglas 45 dient dem Andocken an das hier nicht dargestellte Auge. Die Messeinrichtung 28 wird über ein Koppelelement 30 angekoppelt. Dabei kann das Koppelelement 30 im Fall einer optischen Messeinrichtung 28 (OCT, konfokales Laser-Scanning-System) als optischer Strahlteiler ausgebildet sein. Im Fall einer Ultraschall-Messeinrichtung 28 ist das Koppelelement als akustischer Strahlteiler ausgebildet, wie er bspw. in EP 343 432 A2 beschrieben ist.

Die Messung der Referenzfläche des vorangegangenen Schnittes (primäres Interface) ermöglicht ein exaktes Aufeinandertreffen der alten und neuen Schnitte auch bei Dezentrierung und Verkippung der neuen Behandlung gegenüber der ursprünglichen Behandlung.

Dazu wird beispielsweise eine Aufnahme bzw. Messung der angedockten Cornea während der ursprünglichen Behandlung erzeugt und in einer Datenbank zwischengespeichert. Kommt es nun zu einer Nachbehandlung (Re-Treatment), wird erneut eine Aufnahme bzw. Messung des angedockten Auges erzeugt und mit der gespeicherten Aufnahme bzw. Messung verglichen.

In einer einfachen Ausbaustufe könnte zunächst nur ein Vergleich der alten Schnittführung mit der geplanten neuen Schnittführung erfolgen und das System überprüft damit ob ein Re-Treatment möglich ist. Eine Ausgabe an den Anwender informiert diesen, ob er die Nachbehandlung wie geplant durchführen kann oder nicht.

In einer Ausgestaltung wird die alte Schnittführung in die Berechnung des neuen Schusspatterns einbezogen und Effekte wie oben beschrieben berücksichtigt. Somit ist eine speziell angepasste Behandlung (Customized Treatment) entsprechend der Gegebenheiten in der Cornea des Patienten möglich.

Vorteilhaft erfolgt das Re-Treatment mittels eines Einzelschnittes, da hierbei kein Abstand zum vorherigen Schnitt eingehalten werden muss und damit Hornhautgewebe gespart werden kann und daher nicht so schnell die Grenze von 250 μm Reststromadicke erreicht wird. Aber auch ein erneutes Treatment unterhalb des ersten Schnittes kann mittels einer Registrierung der vorherigen Schnitte verbessert werden, da der Zugangsschnitt (Inzision) zum neuen Lentikel an den alten Zugangsschnitt angesetzt werden kann und keine neue Inzision zur Oberfläche nötig wird. Dies schwächt die Cornea nicht zusätzlich, wie es eine neue Inzision machen würde.

Erfindungsgemäß wird bei der Planung der Lentikelschnittfläche 23 die Dickenänderung der Epithelschicht berücksichtigt. In der ersten Variante der Erfindung erfolgt dies durch die Berücksichtigung der Ergebnisse einer Messung der Lage des ursprünglichen Cap-Schnitts. In einer zweiten Variante der Erfindung wird der Aufwand reduziert, indem die typischen Veränderungen aufgrund der Vorbehandlung anhand von Behandlungsparametern vorausgesagt und für die Nachbehandlung berücksichtigt werden. Denn die Dickenänderung ist für eine Myopiekorrektur mittels SMILE-Verfahren an sich bekannt, bspw. aus N. Luft, M. H. Ring, M. Dirisamer, A. S. Mursch-Edlmayr, T. C. Kreutzer, J. Pretzl, M. Bolz und S. G. Priglinger, "Corneal epthelial remodeling induced by Small Incision Lenticule Extraction (SMILE)," Invest Ophthalmol Vis Sci., Bd. 57, Nr. 9, pp. 176-183, 2016.

Dort wird gezeigt, dass sich die Epithelschicht postoperativ verdickt. Es ist naheliegend, dass das Ausmaß dieser Änderung mit der Art der Änderung und der Korrekturstärke korreliert und die Dynamik mit dem Verlauf des Heilungsprozesses, beginnend mit der Behandlung und zumindest bis zum Erreichen einer langfristig stabilen Refraktion. Nach Luft et al erreicht das Ausmaß der beobachteten Dickenzunahme nach ca. 3 Monaten einen Wert von einigen Mikrometern. Es ist davon auszugehen, dass sich dieser Wert dann kaum noch ändert. Die Daten lassen den Schluss zu, dass die Epitheldickenzunahme beim SMILE-Verfahren zur Myopiekorrektur nahezu gleichmäßig erfolgt und eine Altersabhängigkeit besteht.

$$\textit{Δ}\text{Thickness} = \textit{Δ}\text{Thickness} (\textit{Δ}\text{SE, Age}).$$

$$\textit{Δ}\text{Thickness} = 0.707\mu\text{m} + 1.268\mu\text{m/dpt } \textit{Δ}\text{SE} - 0.038\mu\text{m/(dpt}\cdot\text{y) } \textit{Δ}\text{SE} \cdot (\text{Age-30y}).$$

Falls die Dickenzunahme auf der Oberfläche der Hornhaut ΔThickness(r, φ) ungleichmäßig erfolgt, wie dies in einer etwa zur gleichen Zeit erschienenen Arbeit von in S. Ganesh, "Epithelial Thickness Profile Change Following Small Incision Refractive Lenticule Extraction (SMILE) for Myopia and Myopic Astigmatism," JRS, Bd. 32, Nr. 7, pp. 473-478, 2016 beobachtet wurde, kann sie im Verlauf der Heilung nach einer Behandlung eine größere refraktive Änderung bewirken als wenn sie gleichmäßig erfolgt, wie dies von Luft et al beobachtet wurde. Eine refraktive Änderung könnte sowohl Sphäre und Zylinder als auch Aberrationen höherer Ordnung betreffen. Eine inhomogene Dickenänderung ist bei der Planung der Nachbehandlung aufwändiger zu berücksichtigen.

Auch die WO 2016/144404 A1 weist auf das postoperative Epitheldickenwachstum hin.

Die Schnitte der Nachbehandlung müssen bezüglich der Schnitte der Erstbehandlung korrekt positioniert werden. Dies schließt alle drei Raumrichtungen ein. Zu diesem Zweck berechnet die Vorrichtung eine Standard-Nachbehandlung, basierend auf den Daten der Erstbehandlung, und schlägt diese dem Anwender vor. Durch die Verwendung von Behandlungsdaten der Erstbehandlung nimmt die Planung der Zweitbehandlung Bezug auf das Koordinatensystem der Erstbehandlung. Das Koordinatensystem der Zweitbehandlung wird jedoch in der Regel gegenüber dem der Erstbehandlung zumindest verschoben und / oder verdreht sein. Eine solche Verschiebung ist erfindungsgemäß zu berücksichtigen. Unter Koordinatensystemen werden im Folgenden immer Augenkoordinatensysteme verstanden werden.

Somit können Verschiebungen verschiedene Ursachen haben:

1. Abweichung zwischen Gerätekoordinatensystem und Augenkoordinatensystem bei der Erstbehandlung
2. Abweichung zwischen Gerätekoordinatensystem und Augenkoordinatensystem bei der Zweitbehandlung
3. Abweichung zwischen Gerätekoordinatensystemen der Erst- und Zweitbehandlung
4. Abweichung zwischen Augenkoordinatensystemen der Erst- und Zweitbehandlung Eine erste Variante der Schnittführung sieht vor, dass ein sekundärer Lentikelschnitt 23 unterhalb (posterior) des bereits bestehenden Interfaces (Capschnitt 22 der Erstbehandlung) angelegt wird und durch den Lentikelrandschnitt 25 mit dem bereits existierenden Interface verbunden wird. Dadurch wird ein anschließend manuell extrahierbares Lentikel erzeugt.

Diese Variante ist in Fig. 8 dargestellt, wobei das Dickenwachstum des Epithels 31 vergröbert dargestellt ist

Eine zweite Variante der Schnittführung sieht vor, dass ein sekundärer Lentikelschnitt oberhalb (anterior) des bereits bestehenden Interfaces angelegt wird und durch einen Lentikelrandschnitt mit dem bereits existierenden Interface verbunden wird. Der Lentikelrandschnitt wird bei dieser Variante der Schnittführung vorteilhaft zeitlich vor dem Lentikelschnitt erzeugt. So entsteht wiederum ein manuell extrahierbares Lentikel.

Für beide Varianten der Schnittführung ist es wichtig, dass die neuen Schnitte eine möglichst exakte Lagebeziehung in Bezug auf bereits existierende Schnitte haben. Aufgrund der beschriebenen Verschiebungen der Koordinatensysteme ist eine Vorhersage der lateralen und axialen Position des bereits existierenden Interfaces bei der Zweitbehandlung nur mit einer begrenzten Genauigkeit bekannt. Das kann zu zwei wesentlichen Problemen führen:

1. Das durch das Zusammenwirken von alten und neuen Schnitten erzeugte Lentikel hat nicht die richtige Form für die beabsichtigte refraktive Korrektur. In diesem Fall reduziert die Korrekturabweichung möglicherweise die Wirksamkeit des Verfahrens.
2. Alte und neue Schnitte führen nicht zur vollständigen Separierung eines Lentikels, weil sie sich nicht berühren oder schneiden. In diesem Fall scheitert die Lentikelextraktion möglicherweise ganz.

In einer Ausführungsform der Erfindung wird daher der Randschnitt bei der Zweitbehandlung so verlängert, dass er das bestehende Interface mit Sicherheit kreuzt. Beispielsweise wird der Randschnitt bei der ersten Variante der Schnittführung ins Innere des primären (und sekundären) Caps verlängert, so dass möglichst ein Cross-Cut mit dem bestehenden Interface entsteht, d.h. der Randschnitt 25 schneidet den Capschnitt 22 (in Fig. 8 nicht so dargestellt). Dadurch können axiale Lagefehler ausgeglichen werden. Dies ist besonders wichtig, weil die durch eine Erstbehandlung verursachte Veränderung des Epithels hauptsächlich eine axiale Verschiebung zwischen Augenkoordinatensystemen der Erst- und Zweitbehandlung bewirkt.

In einer anderen Ausführung der Erfindung wird die durch die Veränderung des Epithels verursachte Verschiebung geschätzt und entsprechend kompensiert. Diese Schätzung basiert auf der Information aus der Erstbehandlung, insbesondere der beabsichtigten und/oder erzielten Refraktionsänderung, dem Alter des Patienten und einer Keratometrie. Für die durch die Veränderung des Epithels verursachte Verschiebung kann beispielsweise eine aus Luft et al bekannte Näherungsformel verwendet werden oder eine andere Näherungsformel, die ggf. auch inhomogene Verdickung berücksichtigt. Die Verschiebung des Interfaces gegenüber der in der Erstbehandlung geplanten Lage ist entsprechend berechenbar. Beispielsweise führt eine homogene Verdickung des Epithels letztlich zu einer homogenen Verschiebung des vorhandenen Interfaces bezüglich des Gerätekoordinatensystems für die Nachbehandlung. Diesem Umstand wird bei der Nachbehandlung Rechnung getragen, indem die Lentikelschnittfläche entsprechend verschoben, geneigt oder verbogen wird.

In einer weiteren Ausführung der Erfindung wird die Veränderung des Epithels (Epitheliale Hyperplaie) gemessen und diese Information mit der ebenfalls bekannten Information über die Lage der Schnitte der Erstbehandlung verknüpft und so die Lage der neuen Schnitte entsprechend verändert. Die Messung der Veränderung des Epithels (Epitheliale Hyperplaie) kann beispielsweise mit einem hochauflösenden OCT oder Ultraschall-Messgerät erfolgen. Die Nachbehandlung wird daran angepasst, indem die Lentikelschnittfläche 23 entsprechend gekrümmt und positioniert wird. Insbesondere kann die sekundäre Lentikelfläche, ebenso wie bei einer Primärbehandlung, mit der gewünschten refraktiven Wirkung ausgeführt werden, indem die virtuelle Cap-Fläche der Zweitbehandlung, die bereits als primäres Interface vorhanden ist und daher nicht noch einmal geschnitten wird, für die Berechnung der korrekten Lentikelschnittfläche in Form und Lage mit dem vorhandenen Interface der Erstbehandlung räumlich zur Deckung gebracht wird. Alternativ kann einer quantifizierten Abweichung durch einen entsprechenden Vorhalt bei der Zweitbehandlung entgegengewirkt werden.

In einer weiteren Ausführung der Erfindung wird die durch die Veränderung des Epithels (Epitheliale Hyperplaie) verursachte Verschiebung gemessen und die Lage der neuen Schnitte wird entsprechend korrigiert. Die Messung von Lage und Form des vorhandenen Interfaces kann beispielsweise mit einem hochauflösenden OCT oder Ultraschall-Messgerät erfolgen wie in Fig. 7 dargestellt. Die Nachbehandlung wird daran angepasst, indem die Lentikelschnittfläche 23 entsprechend gekrümmt und positioniert wird. Insbesondere kann die Lentikelfläche, ebenso wie bei einer Primärbehandlung, mit der gewünschten refraktiven Wirkung ausgeführt werden, indem die virtuelle Cap-Fläche der Zweitbehandlung, die bereits als Interface vorhanden ist und daher nicht noch einmal geschnitten wird, für die Berechnung der korrekten Lentikelschnittfläche in Form und Lage mit dem vorhandenen Interface der Erstbehandlung räumlich zur Deckung gebracht wird.

Die beschriebenen Ausführungsformen können einzeln realisiert oder miteinander kombiniert werden. Die Kombination bietet zusätzlich die Möglichkeit zur Berechnung von Abweichungen der verschiedenen Planungsmethoden untereinander. Diese Information kann nicht nur zur Plausibilitätsprüfung verwendet werden. Sondern es können auch cross-cuts abgeleitet werden, deren Ausmaß an die Ungenauigkeit der verschiedenen Berechnungsverfahren angepasst ist.

In einer Weiterentwicklung der Erfindung wird die Lage und Form des aus der Primärbehandlung stammenden Interfaces bezüglich des Koordinatensystems des Behandlungsgerätes berechnet. Dafür reicht es im Falle eines beispielsweise vakuumfixierten Kontaktglases aus, die Tiefe des Interfaces als Funktion des Ortes bezüglich der Oberfläche des Auges zu bestimmen. Dies kann aufwandgering mit einem hochauflösenden OCT erfolgen. Die dann vorliegenden Daten T(r, $\phi$) weisen in der Regel eine Rotationssymmetrie auf und können gut durch ein rotationssymmetrisches Polynom P(r) angenähert werden. Der Lentikelschnitt L(r, $\phi$) wird zunächst beispielsweise bei der ersten Variante der Schnittführung unter der Annahme eines homogenen Caps der Dicke D berechnet und nachfolgend mit der Funktion P(r) - D beaufschlagt. Der so erzeugte Lentikelschnitt L'(r, $\phi$)=L(r, $\phi$)+P(r)-D ist hinsichtlich der durch Epithelisierung (epithelial remodeling) auftretenden Verschiebung des primären Interfaces relativ zur Oberfläche des Auges korrigiert. Dieses Verfahren zur Korrektur nach dem Prinzip einer Störungsrechnung kann durch den Fachmann schrittweise verfeinert werden.

In einer weiteren Ausführungsform kann der Anwender die neuen Schnitte in einem simulierten Querschnittsbild der Cornea angezeigt bekommen und zusätzlich die aus der Erstbehandlung verbliebenen Schnitte. Darüber hinaus wird die vorhandene Information über anatomische Veränderungen zur Berechnung einer Korrektur der Anzeige verwendet. Die Epithelverdickung kann beispielsweise aus der Differenz der prä-operativen Pachymetrie, der Lentikeldicke (bzw. des Lentikelprofils) bei der Erstbehandlung und der aktuellen Pachymetrie abgeleitet werden. Diese einfache Abschätzung kann mit weiterer Information ergänzt werden, etwa einer gemessenen Epitheldicke, einem Epitheldickenprofil, einer subepithelialen Topografie oder einem hochauflösenden Schnittbild (OCT-Bild (B-Scan), Scheimpflug o.ä.) und dem darin identifizierten Interface-Verlauf bezüglich der Vorderseite der Cornea. Die geometrische Unsicherheit der dargestellten Schnitte wird geeignet dargestellt (z.B. Fehlerbalken oder semi-transparente Verbreiterungen eines betroffenen Elements in der Darstellung). Falsche oder kritische Geometrien werden optisch hervorgehoben (z.B. Warnzeichen).

Da die refraktive Wirkung des sekundären Lentikels unmittelbar mit seiner Form im Zusammenhang steht, wirkt sich ein Positionierungsfehler der Zweitbehandlung gegenüber den bereits bestehenden Schnitten auf die refraktive Wirkung des sekundären Lentikels aus. Daher kann in einer Weiterentwicklung der Erfindung die voraussichtliche Brechkraft des Lentikels einschließlich der sich aus der Epitheldickenänderung ergebenden Positionierungsfehler und Varianz der refraktiven Wirkung zur Anzeige gebracht werden. Der Anwender hat dann die Möglichkeit, entsprechende Optimierungen vorzunehmen, um eine Zielrefraktion auch unter Einbeziehung möglicher Fehler optimal zu planen. Beispielsweise kann, auf die vorhandene Akkommodationsfähigkeit des Auges (bei jungen Patienten) Bezug nehmend, eine Verschiebung der Zielrefraktion in den Bereich sehr kleiner Hyperopien erfolgen.

Die Planungseinrichtung visualisiert die Schnittgeometrie in einer virtuellen Darstellung der vorhandenen geometrischen Situation am Auge und der überlagerten Darstellung der geplanten Schnitte. Dabei werden auch Schnitte aus der Erstbehandlung mit angezeigt. In einer Weiterentwicklung wird die Ungenauigkeit der Lage dieser Schnitte geeignet visualisiert (z.B. Fehlerbalken, Halbtransparenz, Scharbild).

Die Planungseinrichtung kann ein Prognosemodell für typische post-operative anatomische Veränderungen nach SMILE beinhalten, die bei der Behandlungsplanung einer SMILE-Nachkorrektur als Ausgangspunkt dienen ("Näherung 1. Ordnung"). Unmittelbar vor der Zweitbehandlung gewonnene Diagnosedaten (Topographie, Wellenfront, Pachymetrie, OCT, Scheimpflug) können zur Verfeinerung der Planung verwendet werden ("Näherung 2. Ordnung"). Intra-operative Diagnosedaten (OCT nach Andocken) können ebenfalls noch hinzugezogen werden. Eventuell dienen die unterschiedlichen Eingangsinformationen auch nur zur Bestätigung der bereits vorgenommenen Planung bzw. zur Fehlerschätzung und Prognose.

Die Prognose der refraktiven Wirkung kann über die Zielrefraktion (Mittelwert) hinausgehen und also auch Verschiebungen und erwartete Streuung umfassen. Dafür können die verschiedenen Ursachen für Abweichungen einbezogen werden.

Zusätzlich sei noch angemerkt, dass die Behandlungsvorrichtung 1 bzw. die Planungseinrichtung 16 natürlich auch die Durchführung des zuvor allgemein erläuterten Verfahrens konkret realisiert.

Eine weitere Ausführungsform der Planungseinrichtung besteht in Form eines Computerprogramms bzw. eines entsprechenden Datenträgers mit einem Computerprogramm, der die Planungseinrichtung auf einem entsprechenden Computer realisiert, so dass die Eingabe der Messdaten über geeignete Datenübertragungsmittel an den Computer erfolgt und die Steuerdaten von diesem Computer an das Steuergerät 11 übertragen werden, wozu wiederum dem Fachmann bekannte Datenübertragungsmittel in Frage kommen.

**Patentansprüche**

1. Planungseinrichtung (16) zum Erzeugen von Steuerdaten für eine Behandlungsvorrichtung (1)

   zur Augenchirurgie, die mittels einer Lasereinrichtung (4) zumindest eine Schnittfläche (22, 23, 24, 25, 26) in der Hornhaut (17) erzeugt, wobei die Planungseinrichtung Berechnungsmittel zum Festlegen von Hornhaut-Schnittflächen, wie Cap-Schnitt (22), Lentikelschnitt (23), (Lentikel-)Randschnitt (25), Zugangs- bzw. Öffnungsschnitt (26, 24), aufweist, wobei die Berechnungsmittel die Hornhaut-Schnittflächen basierend auf den Daten einer Refraktionskorrektur festlegen, wobei die Daten der Refraktionskorrektur auch Daten einer Vorbehandlung beinhalten, und für die Hornhaut-Schnittflächen einen Steuerdatensatz zur Ansteuerung der Lasereinrichtung erzeugen,
   **gekennzeichnet dadurch, dass** die Planungseinrichtung (16) so ausgebildet ist, dass eine Dickenänderung des Epithels (31) bei der Planung berücksichtigt wird, und wobei die Dickenänderung im Wesentlichen ein durch eine Vorbehandlung hervorgerufenes Dickenwachstum ist.

2. Behandlungsvorrichtung (1) zur Augenchirurgie, die

   - eine Lasereinrichtung (4) aufweist, welche mittels Laserstrahlung (6) gemäß Steuerdaten zumindest eine Schnittfläche in der Hornhaut (17) erzeugt, und
   - eine Planungseinrichtung (16) zum Erzeugen der Steuerdaten nach Anspruch 1 aufweist.

3. Verfahren zum Erzeugen von Steuerdaten für eine Behandlungsvorrichtung (1) zur Augenchirurgie, die mittels einer Lasereinrichtung (4) zumindest eine Schnittfläche (22, 23, 24, 25, 26) in der Hornhaut erzeugt, wobei das Verfahren durch folgende Schritte gekennzeichnet ist: Messung der Lage voroperativer Schnitte, Bereitstellen von Hornhaut-Daten, basierend auf Daten einer Refraktionskorrektur, Festlegen von Hornhaut-Schnittflächen, und Erzeugen eines Steuerdatensatzes zur Ansteuerung der Lasereinrichtung zum Schneiden der Hornhaut-Schnittflächen, wobei beim Erzeugen des Steuerdatensatzes eine Dickenänderung des Epithels (31) berücksichtigt wird und die Daten der Refraktionskorrektur auch Daten einer Vorbehandlung beinhalten, und wobei die Dickenänderung im Wesentlichen ein durch eine Vorbehandlung hervorgerufenes Dickenwachstum ist.

4. Verfahren nach Anspruch 3, wobei die Dickenänderung des Epithels (31) mit einer Messvorrichtung bestimmt wird, die vorzugsweise aus der Gruppe Optische Kohärenztomografie (OCT), Scheimpflug-Kamera, Ultraschall-Messsystem und konfokales Laserscanningsystem kommt, und deren Messgenauigkeit gleich oder besser als 2 $\mu m$ ist.

5. Verfahren nach Anspruch 3, wobei die Dickenänderung mittels als Nomogramm, a-priori-Wissen oder Abschätzung vorliegenden Progammcodes bestimmt wird

6. Einrichtung nach einem der Ansprüche 1 oder 2, **gekennzeichnet dadurch, dass** eine Messeinrichtung (28) zur Messung der Epitheldicke vorgesehen ist, welche mit der Planungseinrichtung verbunden ist und vorzugsweise aus der Gruppe Optische Kohärenztomografie (OCT), Scheimpflug-Kamera, Ultraschall-Messsystem und konfokales Laserscanningsystem kommt.

**7.** Einrichtung nach Anspruch 6, **gekennzeichnet dadurch, dass** die Messeinrichtung im Wesentlichen online mit der Planungseinrichtung verbunden ist.

**8.** Einrichtung nach Anspruch 6 oder 7, wobei die Dickenänderung des Epithels mit einer Messvorrichtung bestimmt ist, deren Messgenauigkeit gleich oder besser als 2 $\mu$m ist

**9.** Einrichtung nach einem der Ansprüche 1 oder 2, wobei die Dickenänderung mittels Nomogramm, a-priori-Wissen oder Abschätzung als Progammcode vorliegt.

**10.** Computerprogrammprodukt mit Programm-Code, der bei Ausführung auf einem Computer das Verfahren nach Anspruch 4 oder 5 ausführt.

**11.** Datenträger mit einem Computerprogrammprodukt nach Anspruch 10.

## Claims

**1.** Planning device (16) for creating control data for a treatment apparatus (1) for eye surgery, which creates at least one cut surface (22, 23, 24, 25, 26) in the cornea (17) by means of a laser device (4), wherein the planning device comprises calculation means for defining corneal cut surfaces, such as the cap incision (22), lenticule incision (23), (lenticule) edge incision (25), access or opening incision (26, 24), wherein the calculation means define the corneal cut surfaces on the basis of the data of a refraction correction, with the data of the refraction correction also including data of a pretreatment, and create for the corneal cut surfaces a control data record for controlling the laser device, **characterized in that** the planning device (16) is designed in such a way that a change in thickness of the epithelium (31) is taken into account in the planning, and wherein the change in thickness is essentially a thickness growth caused by a pretreatment.

**2.** Treatment apparatus (1) for eye surgery, which comprises

- a laser device (4) which in accordance with control data creates at least one cut surface in the cornea (17) by means of laser radiation (6), and
- a planning device (16) for creating the control data according to Claim 1.

**3.** Method for creating control data for a treatment apparatus (1) for eye surgery, which by means of a laser device (4) creates at least one cut surface (22, 23, 24, 25, 26) in the cornea, wherein the method is **characterized by** following steps: measuring the position of preoperative incisions, providing corneal data on the basis of data of a refraction correction, defining corneal cut surfaces, and creating a control data record for controlling the laser device for cutting the corneal cut surfaces, wherein a change in thickness of the epithelium (31) is taken into account when creating the control data record, and the data of the refraction correction also include data of a pretreatment, and wherein the change in thickness is essentially a thickness growth caused by a pretreatment.

**4.** Method according to Claim 3, wherein the change in thickness of the epithelium (31) is determined using a measuring apparatus which is preferably from the group of optical coherence tomography (OCT) device, Scheimpflug camera, ultrasonic measuring system and confocal laser scanning system and whose measurement accuracy is equal to or better than 2 $\mu$m.

**5.** Method according to Claim 3, wherein the change in thickness is determined by means of program codes available as nomogram, a-priori knowledge or estimation.

**6.** Device according to either of Claims 1 and 2, **characterized in that** a measuring device (28) is provided for measuring the epithelial thickness and is connected to the planning device and preferably is from the group of optical coherence tomography (OCT) device, Scheimpflug camera, ultrasonic measuring system and confocal laser scanning system.

**7.** Device according to Claim 6, **characterized in that** the measuring device is essentially connected online to the planning device.

**8.** Device according to Claim 6 or 7, wherein the change in thickness of the epithelium is determined using a measuring apparatus whose measurement accuracy is equal to or better than 2 $\mu$m.

9. Device according to either of Claims 1 and 2, wherein the change in thickness by means of nomogram, a-priori knowledge or estimation is present as program code.

10. Computer program product with program code which, upon execution on a computer, carries out the method according to Claim 4 or 5.

11. Data medium having a computer program product according to Claim 10.

**Revendications**

1. Dispositif de planification (16) pour générer des données de commande pour un dispositif de traitement (1) conçu pour la chirurgie oculaire, qui génère au moins une surface d'incision (22, 23, 24, 25, 26) dans la cornée (17) au moyen d'un dispositif laser (4), le dispositif de planification comprenant des moyens de calcul conçus pour définir des surfaces d'incision de la cornée, comme une incision antérieure (22), une incision de lenticule (23), une incision de bord (de lenticule) (25), une incision d'accès ou d'ouverture (26, 24), les moyens de calcul établissant les surfaces d'incision de la cornée sur la base des données d'une correction de la réfraction, les données de correction de la réfraction contenant également des données d'un prétraitement, et générant un jeu de données de commande permettant de commander le dispositif laser pour les surfaces d'incision de la cornée, **caractérisé en ce que** le dispositif de planification (16) est conçu de telle sorte qu'une modification de l'épaisseur de l'épithélium (31) soit prise en compte dans la planification, et la modification de l'épaisseur étant essentiellement une croissance de l'épaisseur due à un prétraitement.

2. Dispositif de traitement (1) destiné à la chirurgie oculaire, lequel comporte

   - un dispositif laser (4) qui génère au moins une surface d'incision dans la cornée (17) à l'aide d'un rayonnement laser (6) en fonction de données de commande, et
   - un dispositif de planification (16) conçu pour générer les données de commande selon la revendication 1.

3. Procédé pour générer des données de commande destinées à un dispositif de traitement (1) conçu pour la chirurgie oculaire, qui génère au moins une surface d'incision (22, 23, 24, 25, 26) dans la cornée au moyen d'un dispositif laser (4), le procédé étant **caractérisé par** les étapes suivantes : la mesure de la position d'incisions préopératoires, la fourniture de données cornéennes sur la base de données de correction de la réfraction, l'établissement de surfaces d'incision de la cornée et la génération d'un jeu de données de commande destinées à piloter le dispositif laser pour la découpe des surfaces d'incision de la cornée, une modification de l'épaisseur de l'épithélium (31) étant prise en compte lors de la génération du jeu de données de commande, les données de correction de la réfraction contenant également des données d'un prétraitement, et la modification de l'épaisseur étant essentiellement une croissance d'épaisseur due à un prétraitement.

4. Procédé selon la revendication 3, **caractérisé en ce que** la modification de l'épaisseur de l'épithélium (31) est déterminée à l'aide d'un dispositif de mesure qui appartient de préférence au groupe comprenant la tomographie par cohérence optique (OCT), une caméra de Scheimpflug, un système de mesure à ultrasons et un système de balayage laser confocal, et dont la précision de mesure est égale ou supérieure à 2 $\mu$m.

5. Procédé selon la revendication 3, dans lequel la modification de l'épaisseur est déterminée au moyen de codes de programme se présentant sous la forme d'un nomogramme, d'une connaissance a priori ou d'une estimation.

6. Dispositif selon l'une quelconque des revendications 1 ou 2, **caractérisé en ce qu'**il est prévu un dispositif de mesure (28) conçu pour mesurer l'épaisseur de l'épithélium, qui est relié au dispositif de planification et appartient de préférence au groupe comprenant la tomographie par cohérence optique (OCT), une caméra de Scheimpflug, un système de mesure à ultrasons et un système de balayage laser confocal.

7. Dispositif selon la revendication 6, **caractérisé en ce que** le dispositif de mesure est pour l'essentiel connecté en ligne au dispositif de planification.

8. Dispositif selon la revendication 6 ou 7, **caractérisé en ce que** la modification de l'épaisseur de l'épithélium est déterminée à l'aide d'un dispositif de mesure dont la précision de mesure est égale ou supérieure à 2 $\mu$m.

**9.** Dispositif selon l'une quelconque des revendications 1 ou 2, **caractérisé en ce que** la modification de l'épaisseur se présente sous la forme d'un code de programme au moyen d'un nomogramme, d'une connaissance a priori ou d'une estimation.

**10.** Produit de programme informatique comprenant un code de programme qui, lorsqu'il est exécuté sur un ordinateur, met en œuvre le procédé selon la revendication 4 ou 5.

**11.** Support de données comprenant un produit de programme informatique selon la revendication 10.

Fig.1

Fig.2

Fig.4

Fig.3

Fig.5

Fig. 6 (a)

Fig. 6 (b)

4

5

28

30

45

6

Fig. 7

31

22

17

26

21

23

25

Fig. 8

Fig. 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102007019814 A1
- DE 102012022081 A1
- US 2011224658 A1
- DE 102005040338 A1
- DE 69500997 T2
- DE 102007019813 A1
- DE 10323422 A1
- EP 343432 A2
- WO 2016144404 A1

**In der Beschreibung aufgeführte Nicht-Patentliteratur**

- Very high frequency digital ultrasound: artemis 2 scanning in corneal refractive surgery. **PAOLO VINCIQUERRA** ; **FABRIZIO CAMESASCA** ; **DAN Z. REINSTEIN** ; **RONALD H. SILVERMAN** ; **TIMOTHY J. ARCHER**. Refractive Surface Ablation: PRK, LASEK, Epi-lasik, Custom, PTK, and Retreatment. SLACK Inc., 2007

- **N. LUFT** ; **M. H. RING** ; **M. DIRISAMER** ; **A. S. MURSCH-EDLMAYR** ; **T. C. KREUTZER** ; **J. PRETZL** ; **M. BOLZ** ; **G. PRIGLINGER**. Corneal epthelial remodeling induced by Small Incision Lenticule Extraction (SMILE). *Invest Ophthalmol Vis Sci.*, 2016, vol. 57 (9), 176-183
- **S. GANESH**. Epithelial Thickness Profile Change Following Small Incision Refractive Lenticule Extraction (SMILE) for Myopia and Myopic Astigmatism. *JRS*, 2016, vol. 32 (7), 473-478